# EUROPÄISCHE PATENTSCHRIFT

(11) **EP 0 842 442 B1**
(45) Veröffentlichungstag und Bekanntmachung des Hinweises auf die Patenterteilung: **19.09.2001**
(21) Anmeldenummer: 96924778.2
(22) Anmeldetag: 25.07.1996
(51) Int. Cl.: G01T 5/08

(54) **VERFAHREN UND VORRICHTUNG ZUR MESSUNG DER STRAHLUNGSTIEFE EINER STRAHLUNG**
PROCESS AND DEVICE FOR MEASURING THE RADIATION DEPTH OF RADIATION
PROCEDE ET DISPOSITIF POUR MESURER LA PROFONDEUR D'UN RAYONNEMENT

(30) Priorität: 01.08.1995 DE 19528096
(43) Veröffentlichungstag der Anmeldung: 20.05.1998
(73) Patentinhaber: FORSCHUNGSZENTRUM JÜLICH GMBH, 52425 Jülich (DE)
(72) Erfinder: GRIPP, Stefan, D-40764 Langenfeld (DE); HÄSING, Friedrich-Wolfgang, D-52428 Jülich (DE); BÜKER, Harald, D-52428 Jülich (DE)
(86) Internationale Anmeldenummer: DE9601405
(87) Internationale Veröffentlichungsnummer: WO9705506

(56) Entgegenhaltungen:
- EP-A- 0 416 493
- GB-A- 2 200 984
- IEE PROCEEDINGS -J, Bd. 140, Nr. 4, August 1993, STEVENAGE GB, Seiten 267-272, XP002016119 VASSILOPOULOS, C ET AL.: "conception of an ionising radiation detection scheme based on controlled light induced annealing of silica fibres"

## Beschreibung

Die Erfindung bezieht sich auf ein Verfahren zur Messung der Strahlungstiefe einer Strahlung sowie auf eine Meßeinrichtung, bestehend aus zwei Sensoren zur Messung von Strahlendosen.

Es ist bekannt, die Energie einer Elektronenstrahlung durch Vermessung der Eindringtiefe der Strahlung in ein Objekt mit einer lonisationskammer bzw. mit einer Anordnung von mehreren lonisationskammern zu ermitteln. Dieses Verfahren ist zeitraubend und meßtechnisch aufwendig.

Eine Meßeinrichtung, bestehend aus zwei Sensoren zur Messung von Strahlendosen, ist aus der deutschen Patentanmeldung, Aktenzeichen 195 03 647.6-33 bekannt. Diese ist jedoch nicht für die Messung der Eindringtiefe, sondern für eine Messung einer gewebeäquivalenten Dosis vorgesehen.

Aufgabe der Erfindung ist die Schaffung eines Verfahrens zur schnellen und einfachen Messung der Eindringtiefe einer (ionisierenden) Strahlung sowie die Schaffung einer Vorrichtung zur Durchführung des Verfahrens.

Gelöst wird die Aufgabe durch ein Verfahren sowie eine Vorrichtung mit den Merkmalen des Verfahrens- bzw. des Vorrichtungsanspruchs.

Zur Durchführung des Verfahrens wird die Strahlendosis (in einem Objekt) entlang des Strahlengangs bis zur Eindringtiefe gemessen. Es reicht aus, die Messung auf eine Wegstrecke am Ende der Strahlung zu beschränken. Es ist also nicht erforderlich, die Strahlung vollständig zu erfassen.

Ferner wird die Strahlendosis in einem innerhalb des Strahlengangs befindlichen Volumenelement gemessen. Um keine Artefakte aufgrund zeitlicher Intensitätsschwankungen der Strahlung zu erhalten, ist es vorteilhaft, diese und die vorbeschriebene Messung simultan durchzuführen.

Vorteilhaft werden die Messungen mit gleichen Sensoren durchgeführt, um durch Quotientenbildung einen dosisunabhängigen Wert zu erhalten. Andernfalls müssen Näherungen in Kauf genommen werden, oder es müssen Ausgleichsrechnungen vorgenommen werden.

Im Anschluß an die beiden Messungen wird ein dosisunabhängiger Quotient aus beiden Meßwerten gebildet.

Anhand von Referenzwerten wird schließlich die Eindringtiefe der Strahlung in das Objekt ermittelt.

Die Eindringtiefe ist ein Maß für die Energie der Strahlung. Folglich ermöglicht das Verfahren die Bestimmung der Energie eines Strahlenbündels von hochenergetischer Korpuskularstrahlung (z.B. Elektronen, Protonen, Ionen, Neutronen). Das Verfahren eignet sich unter anderem für die routinemäßige Überprüfung der Energie von bei der Strahlentherapie eingesetzter Elektronenstrahlung im Energiebereich zwischen 2 und 25 MeV.

Eine Meßeinrichtung zur Durchführung des Verfahrens besteht aus zwei Sensoren (1), (2) zur Messung von Strahlendosen. Diese weisen eine räumliche Ausdehnung auf, die parallel zu oder entlang einer geraden Linie angeordnet sind. Des weiteren deckt der eine ("lange") Sensor (2) eine längere Strecke entlang der Linie ab als der andere ("kurze") Sensor (1).

Ziel dieser Anordnung ist, daß Strahlung, die parallel zu Linie verläuft, bei geeignet im Strahlengang positionierter Vorrichtung durch die Sensoren registriert wird. Wesentlich ist dann, daß der lange Sensor (2) im Unterschied zum kurzen Sensor (1) die Strahlung bis zu ihrer Eindringtiefe registriert. Ferner muß dann zwischen dem kurzen Sensor (1) und dem langen Sensor (2) bei geeigneter Positionierung im Strahlengang ein ausreichend unterschiedliches Sensorsignal erzielt werden können. Entsprechend diesen Anforderungen ist der Längenunterschied bzw. Unterschied bezüglich der abgedeckten Strecke parallel zur Linie zu wählen. Mit großen Unterschieden können die Anforderungen erfüllt werden. Es hat sich gezeigt, daß z. B. eine Längendifferenz von wenigstens 20 mm (insbesondere bei einer Elektronenstrahlung) vorhanden sein sollte, um die vorgenannte Anforderung zu erfüllen. Kürzere Längenunterschiede sind nur bei hoher Auflösung der Sensoren möglich. Zweckmäßigerweise beträgt der Längenunterschied 40 mm und mehr.

Vorzugsweise deckt der kurze Sensor (1) einen Bereich von weniger als 10 mm Tiefe ab und der "lange" Sensor einen Bereich von wenigstens 30 mm. Ein derartig dimensionierte: Sensor kann problemlos in erforderlicher Weise im Strahlengang positioniert werden.

Als Sensoren eignen sich strahlungsempfindliche Lichtwellenleiter, szintillierende Lichtwellenleiter, Ionisationskammern usw..

Handelt es sich um hochempfindliche Lichtwellenleiter (z. B. PbO-Fasern, dotiert mit Materialien, die eine hohe effektive Ordnungszahl aufweisen), so kann der eine Lichtwellenleiter parallel zum Strahlengang und der andere beliebig (insbesondere senkrecht zum Strahlengang) angeordnet sein.

Werden Lichtwellenleiter mit geringer Nachweisempfindlichkeit eingesetzt, so ist es vorteilhaft den langen Sensor zwecks Kompensation seiner geringen

Empfindlichkeit insbesondere spiral- oder zickzackförmig um die Linie "gewickelt" anzuordnen. Dabei gilt: je dichter die Wicklung, um so größer die Meßempfindlichkeit. Allgemein sollte ein Sensor bei geringer Empfindlichkeit zwecks Kompensation dieses Mankos derart angeordnet sein, daß er zusätzlich zu seiner Längsführung noch eine große Querschnittsfläche abdeckt.

Es zeigen:
- Figur 1:: Ausführungsbeispiel einer Meßeinrichtung.
- Figur 2:: verfahrensgemäß ermittelte Ergebnisse.

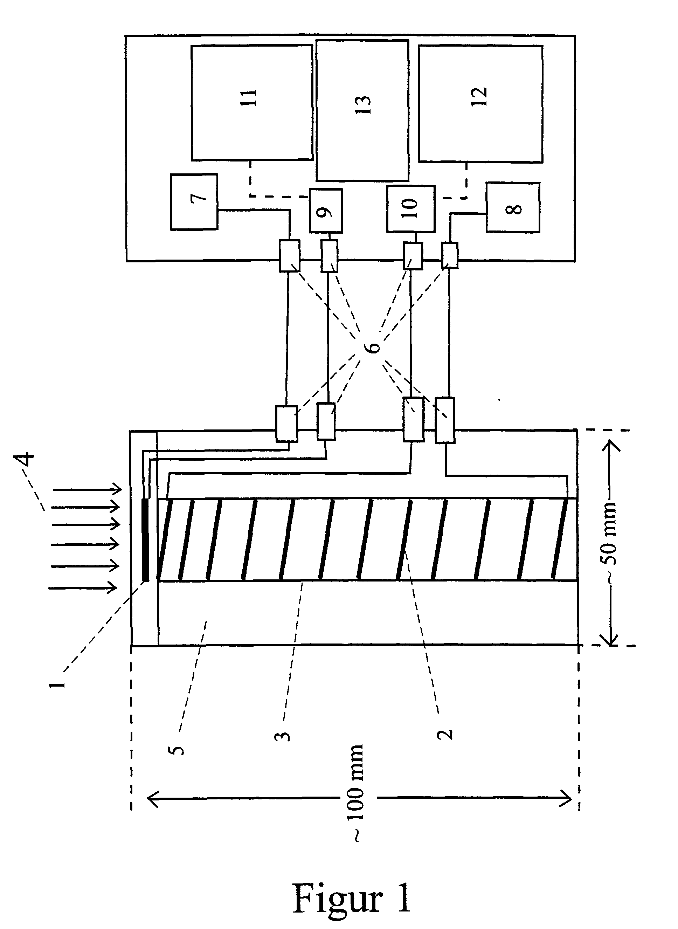

Ein strahlungsempfindlicher Lichtwellenleiter 1 in Figur 1 ist in einer Ebene um einen Plexiglaszylinder 3 und ein strahlungsempfindlicher Lichtwellenleiter 2 ist spiralförmig um diesen Plexiglaszylinder 3 gewickelt.

Infolge der Wicklung des Lichtwellenleiters 1 in einer Ebene hängt sein Meßsignal nicht oder nur wenig von der Energie der ionisierenden Strahlung ab, wenn der Plexiglaszylinder 3 parallel zur Strahlung 4 positioniert ist. Lichtwellenleiter 2 erstreckt sich dann in die Tiefe des Meßvolumens (Zylinder 3 und 5), so daß bei ihm die durch die Strahlung induzierte Dämpfung stark von der Eindringtiefe und damit von der Energie der Strahlung abhängt. Der Plexiglaszylinder 3 weist einen Durchmesser von weniger als 25 mm und eine Länge von 100 mm auf. Er befindet sich in einem weiteren äußeren Plexiglaszylinder 5 mit einem Durchmesser von ungefähr 50 mm. Durch Steckverbindungen 6 sind die Lichtwellenleiter 1 und 2 an eine Auswerteeinheit angeschlossen.

Die Auswerteeinheit besteht aus den Lichtquellen (LED) 7 und 8, den Detektoren 9 und 10, den Einrichtungen 11 und 12 zur Ermittlung der Dämpfung sowie einer Einrichtung 13 zur verfahrensgemäßen Berechnung der Eindringtiefe oder Strahlungsenergie. Das Licht der Lichtquelle 7 (bzw. 8) gelangt zum Lichtwellenleiter 1 (bzw. 2), durchläuft diesen und endet schließlich im Detektor 9 (bzw. 10). Mittels des Detektors 9 (bzw. 10) wird die ankommende Lichtintensität gemessen und an die Einrichtung 11 (bzw. 12) weitergeleitet. Mittels der Einrichtung 11 (bzw. 12) wird die Veränderung der Transmission infolge der in den Lichtwellenleiter 1 (bzw. 2) einfallenden Strahlung, also die Dämpfung festgestellt. Mittels der Einrichtung 13 wird der Quotient aus beiden Dämpfungswerten der Einrichtungen 11 und 12 gebildet und hieraus die Eindringtiefe bzw. die Strahlungsenergie ermittelt.

Für die Durchführung des Verfahrens wird die Meßeinrichtung im Strahlengang geeignet positioniert, d. h. die Spirale des Lichtwellenleiters 2 verläuft entlang des Strahlengangs mindestens bis zum Ende der Eindringtiefe. Wird beispielsweise eine Eindringtiefe in der Größenordnung von 70 mm in das Objekt erwartet, so sollte die Spirale mehr als 70 mm tief (z. B. 100 mm) in das Objekt hineinreichen.

Die Transmissionseigenschaften der Lichtwellenleiter verändern sich bei einfallender Strahlung infolge von strahlungsinduzierten Farbzentren im Material der Lichtwellenleiter. Folglich verändern sich die Transmissionseigenschaften der Lichtwellenleiter in Abhängigkeit von Eindringtiefe der Strahlung in Materie (z.B. Plexiglas).

Um die Meßeinrichtung wiederholt einsetzten zu können, müssen die Lichtwellenleiter nach Erreichen ihrer maximalen Dosisbelastung durch Ausheilen der strahlungsinduzierten Farbzentren regeneriert werden. Dieses kann durch optische oder thermische Anregung der in den Störniveaus eingegangenen Elektronen erfolgen.

Die Unabhängigkeit der Sensorsignale von der eingestrahlten Dosis, von der Sensortemperatur und von Fadingeffekten werden erreicht, indem die im Lichtwellenleiter 2 induzierte Dämpfung auf die induziert Dämpfung des Lichtwellenleiters 1 bezogen wird. Bestehen beide Sensorelemente aus dem gleichen Material, so lassen sich durch einfache Quotientenbildung Störeffekte eliminieren.

Die Auflösung und Meßempfindlichkeit der Meßvorrichtung kann durch Wahl der geometrischen Anordnung in weiten Bereichen variiert werden. Dicht (spiralförmig) gewickelt Lichtwellenleiter sorgen beispielsweise für eine hohe Meßempfindlichkeit

Andere geometrische Anordnungen (auch Mehrfachanordnungen zur Bestimmung der radialen bzw. räumlichen Verteilung der Elektronenenergie) sind möglich.

Die Auswertung der Detektorsignale kann sowohl analog als auch numerisch erfolgen. Bei der analogen Signalauswertung können auftretende Nichtlinearitäten in der dargestellten geometrischen Anordnung durch die Geometrie der Wicklung beseitigt werden. Bei der digitalen Auswertung können solche Nichtlinearitäten durch eine im Mikroprozessor abgespeicherte Kalibrierkurve berücksichtigt werden.

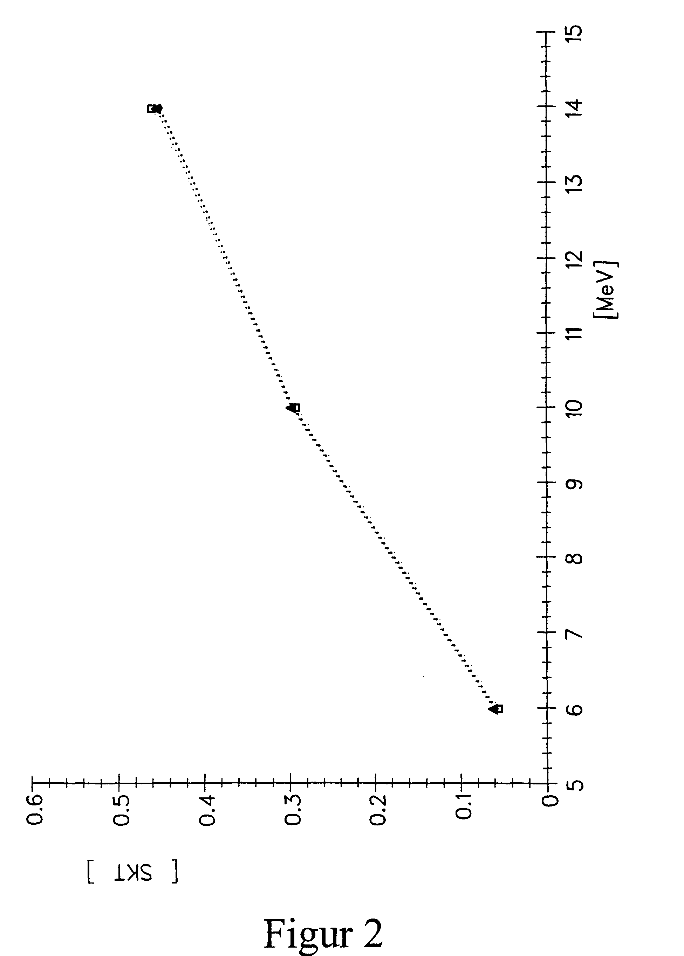

Figur 2 zeigt verfahrensgemäß ermittelte Ergebnisse. Zunächst wurde die strahlungsinduzierte Dämpfung eines 100 mm langen PbO-Sensors gemessen. Es wurden Versuche durchgeführt, bei denen der Sensor 28 bis 82 mm in Plexiglas hineinreichte. Hiermit wurde eine Elektronenstrahlung registriert, deren Energie zwischen 6 und 14 MeV lag. Anschließend wurde mit dem gleichen (regenerierten) Sensor die Dämpfung in 10 mm Tiefe gemessen. Jede Messungen wurden 30 Sekunden lang durchgeführt. Das Diagramm (Skalenteile SKT aufgetragen gegen Energie in MeV) zeigt das Ergebnis der Quotientenbildung.

## Patentansprüche

1. Verfahren zur Messung der Strahlungstiefe einer Strahlung (4) mit folgenden Schritten
a)Messung der Strahlendosis entlang der Strahlung bis zu ihrer Eindringtiefe,
b)Messung der Strahlendosis in einem innerhalb des Strahlengangs befindlichen Volumenelement
c)Bildung eines dosisunabhängigen Quotienten aus beiden Meßwerten,
d)Ermittlung der Strahlungstiefe aus dem Quotienten anhand von Referenzwerten.

2. Einrichtung zur Messung der Strahlungstiele einer Strahlung, bestehend aus zwei Sensoren zur Messung von Strahlendosen,
**dadurch gekennzeichnet, daß**
beide Sensoren eine räumliche Ausdehnung parallel zu oder entlang einer geraden Linie derart aufweisen, daß der eine Sensor (2) eine längere Strecke entlang der Linie abdeckt als der andere Sensor (1).

3. Einrichtung nach vorhergehendem Anspruch,
**dadurch gekennzeichnet, daß**
der Unterschied bezüglich der abgedeckten Strecke wenigstens 20 mm beträgt.

## Claims

1. A method of measuring the radiation depth of a radiation (4), said method consisting of the following steps:
a) measuring the radiation dose along the radiation path up to its depth of penetration,
b) measuring the radiation dose in a volume element located within the radiation path,
c) forming a dose-independent quotient from the two measurement values,
d) determining the radiation depth from the quotient on the basis of reference values.

2. A device for measuring the radiation depth of a radiation, said device consisting of two sensors for measuring radiation doses,
**characterized in that**
the two sensors have a spatial dimension parallel to or along a straight line such that one sensor (2) covers a longer distance along the line than the other sensor (1).

3. A device according to the preceding claim,
**characterized in that**
the difference with regard to the distance covered amounts to at least 20 mm.

## Revendications

1. Procédé de mesure de la profondeur d'un rayonnement (4) comprenant les stades suivants
a) mesure de la dose de rayonnement le long du rayonnement jusqu'à sa profondeur de pénétration,
b) mesure de la dose de rayonnement dans un élément de volume se trouvant dans le trajet du rayonnement,
c) formation d'un quotient indépendant de la dose à partir des deux valeurs de mesure,
d) détermination de la profondeur du rayonnement à partir du quotient au moyen de valeurs de référence.

2. Dispositif de mesure de la profondeur d'un rayonnement constitué de deux capteurs de mesure de doses de rayonnement,
**caractérisé en ce que**
les deux capteurs ont une étendue dans l'espace parallèlement à une ligne droite ou le long d'une ligne droite, de façon que l'un des capteurs (2) recouvre une plus grande étendue le long de la ligne que l'autre capteur (1).

3. Dispositif suivant la revendication précédente, **caractérisé en ce que** la différence entre les étendues recouvertes est d'au moins 20 mm.
